# EUROPEAN PATENT SPECIFICATION

(11) **EP 3 768 773 B1**
(45) Date of publication and mention of the grant of the patent: **18.06.2025**
(21) Application number: 19771349.8
(22) Date of filing: 21.03.2019
(51) Int. Cl.: C08L 1/28, A61K 47/38, A61K 9/48, B29C 41/14, B65D 85/00, C08L 1/02

(54) **VAPORIZER CAPSULES AND METHODS OF MANUFACTURE**
VERDAMPFERKAPSELN UND VERFAHREN ZU IHRER HERSTELLUNG
CAPSULES DE VAPORISATEUR ET PROCÉDÉS DE FABRICATION

(30) Priority: 21.03.2018 US 201862646267 P
(43) Date of publication of application: 27.01.2021
(73) Proprietor: Altria Client Services LLC, Richmond, VA 23230 (US)
(72) Inventor: SELBY, Ryan Daniel, Vancouver, BC V5W 1T2 (CA); KARKAIRAN, Ryan, Vancouver, BC V5V 4P7 (CA)
(74) Representative: Marks & Clerk LLP
(86) International application number: PCT/CA2019/050352
(87) International publication number: WO 2019/178698

(56) References cited:
- EP-A1- 2 078 042
- EP-A1- 2 800 585
- EP-B1- 2 078 042
- WO-A1-2013/009253
- GB-A- 1 144 225
- GB-A- 1 507 141

## Description

### FIELD

Embodiments of the invention generally relate to capsules for storing consumable materials therein, and more particularly to methods of manufacturing the same.

### BACKGROUND

Capsules are a common form of temporarily storing consumable materials. In certain instances, capsules can be used to temporarily store pharmaceuticals for later ingestion by patients. In other instances, capsules can be used as a delivery system for chemical compounds. WO 2013/009253 describes barriers for use in the area of controlled release. EP2078042 provides a composition for the manufacture of hard hydroxypropyl methyl cellulose capsules. GB1507141 provides a method of producing shaped gelatin parts. GB1144225 provides a process for preparing medicinal capsule shells. In yet, other instances, capsules can be used to store vaporizable materials to be vaporized in commercially available vaporizers.

Vaporizer capsules are typically adapted to store vaporizable materials therein, and are typically placed into a vaporizer to vaporize any active ingredients in the vaporizable materials to produce vaporized active ingredients. The vaporized active ingredients, in the form of a vapor, can then inhaled by the user, typically for medical and/or recreational use.

The capsules storing the vaporizable materials therein are typically manufactured with materials that have a boiling temperature sufficiently high enough that the structural integrity of the capsule will not be effected or otherwise compromised when a user vaporizes the active ingredients. Most often, such capsules are manufactured using glass, metal or high temperature resistant plastics.

Recently, in response to environmental concerns, such capsules are being manufactured to incorporate biodegradable and/or compostable properties. For example, the capsules can be made from various forms of biodegradable materials.

There are many prior art examples of producing capsules using traditional film-forming materials or combinations thereof. However, there are no prior art examples of using biodegradable traditional film-forming materials to produce capsules (or structures or shells) with increased thermal resistance such that they may be used as components in vaporization systems employing temperatures between 100°C - 400°C.

The traditional clear biodegradable film materials may include, but are not limited to animal-derived film-forming polymers such as gelatin; non-animal-derived film-forming polymers such as polyvinyl alcohol; plant-derived or bacterial-derived film-forming polymers such as starch, starch derivatives, cellulose, cellulose derivatives such as hydroxypropyl-methyl cellulose (HPMC) and hydroxypropyl cellulose (HPC), and other forms of modified cellulose; or exo-polysaccharides such as xanthan, acetan, gellan, welan, rhamsan, furcelleran, succinoglycan, scleroglycan, schizophyllan, tamarind gum, curdlan, pullulan, dextran and mixtures thereof. These ingredients may be used independently or in any combination thereof.

However, the structural integrity of traditional biodegradable materials becomes compromised when exposed to temperatures sufficiently high enough to vaporize common vaporizable materials and the active ingredients therein. More particularly, the capsules typically melt, burn, become brittle or otherwise breakdown when exposed to routine operating temperatures for vaporizers.

### SUMMARY

While developing and designing products related to the vaporization of consumable substances, it became apparent that it would be desirable to utilize a biodegradable film-forming material for certain components in the design of the vaporization system, namely the capsule for storing vaporizable materials therein and certain tubes and other structures used in the construction of vaporizable capsules or products.

As typical vaporization systems operate at elevated temperatures (between 100°C and 400°C) it would be advantageous that the biodegradable film-forming materials would be able to withstand the elevated operating temperatures. Thus a search began to identify any suitable biodegradable film-forming materials currently in existence. After an extensive search, it was determined that none of the available materials were capable of optimal performance at the temperatures required.

Vaporizer capsules can incorporate thermal resistance properties by combining microfibrillated cellulose (MFC), nanofibril cellulose (NFC), nanocellulose, cellulose nanocrystals (CNC), nanocrystalline cellulose (NCC) or nanofibril cellulose whiskers with traditional biodegradable film-forming materials. The present disclosure can produce capsules with increased thermal resistance properties which may be used effectively as components in vaporization systems operating at temperatures ranging from 100°C - 400°C.

The resulting capsules may be additionally modified to change the color or opacity of the capsules, or printing or other information may be added to the capsules.

In a broad aspect of the invention, a capsule forming composition for manufacturing a capsule comprises a mixture of hydroxypropyl-methyl-cellulose and cellulose nanocrystals in accordance with the claims appended hereto.

In another broad aspect of the invention, a method for manufacturing a capsule comprises providing an elastomeric dipping in having an annular cavity, providing a supporting pin, positioning the supporting pin within the annular cavity of the dipping pin, depositing at least one layer of a capsule forming composition onto an exterior surface of the dipping pin, curing the at least one layer of capsule forming composition for forming a capsule, and removing the formed capsule from the dipping pin in accordance with the claims appended hereto.

In another broad aspect of the invention, a molding pin for manufacturing a capsule from a capsule forming composition comprises a dipping pin having an elastomeric portion on which the capsule forming composition is deposited thereon in accordance with the claims appended hereto.

### BRIEF DESCRIPTION OF THE DRAWINGS

Figure 1 is a flow chart illustrating a prior art method of forming a capsule using known dip molding techniques;

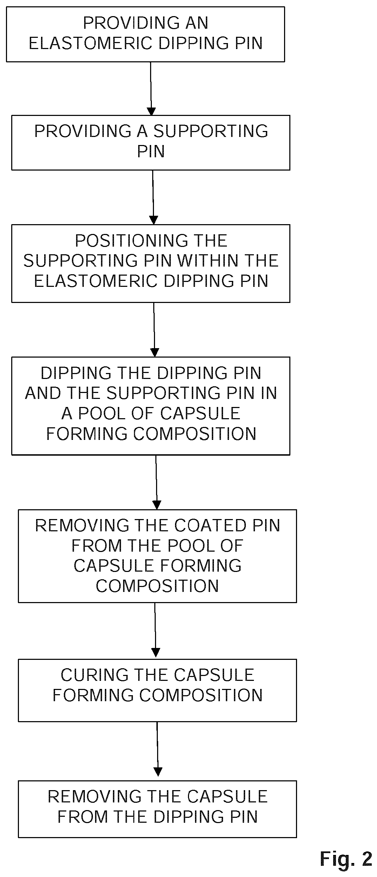

Figure 2 is a flow chart of an embodiment of the present invention illustrating a method for manufacturing a capsule;
Figure 3 is a side elevation view of an embodiment of the invention illustrating a support pin positioned within an elastomeric dipping pin;
Figure 4 is a side cross-sectional view of the embodiment in accordance with Fig. 3;
Figure 5 is a plan view of the embodiment in accordance with Fig. 3, illustrating lubricant grooves disposed within a concentric annular cavity in the elastomeric dipping pin;
Figure 6 is a side elevation view of an embodiment of the invention illustrating a support pin having lubricant grooves disposed on its exterior surface, the support pin positioned within an elastomeric dipping pin; and
Figure 7 is a side cross-sectional view of the embodiment in accordance with Fig. 5.

### DETAILED DESCRIPTION

Using the identified materials above, attempts to manufacture vaporizable capsules using traditional methods, such as dip molding, were made.

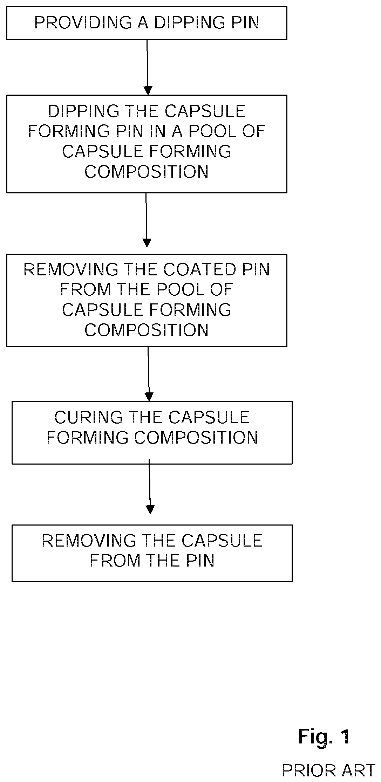

As shown in Fig. 1, traditional methods for forming capsules (such as dip molding) involves dipping or at least partially submerging a capsule forming pin (or dipping pin) in a pool of a capsule forming material or composition to coat the pin, removing the coated pin from the pool, and allowing the capsule forming material to cure on the pin. Once the curing process is completed, the formed capsule can then be removed from the pin for use by gripping the formed capsule on the outside of the formed capsule and applying linear forces to encourage separation of the formed capsule from the dipping pin.

In an embodiment, a formed capsule should be able to withstand heat applied to the capsule during vaporization, and should have sufficient structural integrity for repeated use, and should optionally be biodegradable. Applicant found that creating a capsule using a cellulose product, such as those discussed above, provided a biodegradable and/or compostable capsule, but having differing physical characteristics, depending on the specific cellulose material used. For example, a capsule formed using certain grades of 100% HPMC (hydroxypropyl-methyl cellulose) forms a capsule that can withstand high temperatures, but is malleable and does not have structural integrity. In other words, the capsules formed using greater amounts of HPMC can withstand high temperatures, but are too soft and can easily deform when subjected to the temperatures required for vaporization. In another example, a capsule formed using 100% CNC (cellulose nanocrystals) provides increased structural integrity, but is too brittle, especially when subjected to high temperatures.

Many of the traditional film-forming materials listed above were combined and/or mixed in an effort to produce a composition which would produce a capsule having desirable characteristics. After numerous attempts, a composition comprising a combination of HPMC with cellulose nanocrystals (CNC) resulted in a film which was able to withstand the required operating temperatures of typical vaporizers and still have sufficient structural integrity during and after the heating process. Further, the resulting capsule forming compositions or films had improved strength and other characteristics that were not possessed by either of the materials on their own. In experiments, combining other traditional film-forming materials with CNC (or other forms of modified cellulose such as microfibrillated cellulose (MFC), nanofibril cellulose (NFC), nanocellulose, nanocrystalline cellulose (NCC) or nanofibril cellulose whiskers) met with limited positive results. Further experimentation with different combinations of traditional film-forming materials and CNC (or other forms of modified cellulose such as microfibrillated cellulose (MFC), nanofibril cellulose (NFC), nanocellulose, nanocrystalline cellulose (NCC) or nanofibril cellulose whiskers) also led to limited success.

Experimentation with differing ratios between CNC (or other forms of modified cellulose such as microfibrillated cellulose (MFC), nanofibril cellulose (NFC), nanocellulose, nanocrystalline cellulose (NCC) or nanofibril cellulose whiskers) and traditional film-forming materials were conducted to arrive at optimal ratios. The results of the experiments assisted in demonstrating that the ratios of CNC (or other forms of modified cellulose such as microfibrillated cellulose (MFC), nanofibril cellulose (NFC), nanocellulose, nanocrystalline cellulose (NCC) or nanofibril cellulose whiskers) to the other materials has effects on the thermal resistance of the resulting capsules.

Based on multiple and repeated experiments, Applicant believes that a ratio of 80% HPMC and 20% CNC (or other forms of modified cellulose such as microfibrillated cellulose (MFC), nanofibril cellulose (NFC), nanocellulose, nanocrystalline cellulose (NCC) or nanofibril cellulose whiskers) provides the best results in terms of providing structural integrity and being able to withstand sufficiently high temperatures required for vaporization. Further experimentation with adding different plasticizers, gelling agents, and other materials has also resulted in improved overall characteristics of the capsules.

While the composition of 80% HPMC and 20% CNC (4:1 ratio of HPMC to CNC) provides the most desirable physical characteristics, Applicant found that such a composition was too fluid (having low viscosity) during the dip molding process. That is, after the capsule forming pin or dipping pin is at least partially submerged into a pool of the capsule forming composition for depositing the composition onto the dipping pin, and extracted or pulled out, the composition's fluidity was sufficiently fluid enough to permit pooling, dripping and/or sloughing during the curing process to produce a capsule having a capsule wall that was not uniform in thickness. More specifically, the capsule wall would have a top portion with thickness less than a thickness of a bottom portion of the capsule wall. That is, the top portion of the capsule wall was thinner than a bottom portion of the capsule wall because the composition would flow, drip and/or slough downward (due to gravitational forces) during the curing process.

In order to increase the viscosity of the capsule forming composition, Applicant introduced various gelling, setting and/or curing agents to the composition to encourage and/or expedite the curing process. For example, the addition of small amounts of gellan in combination with elevated temperatures of the dipping pool and lower temperatures during the curing or drying process provided an effective method for sufficiently reducing the viscosity of the capsule forming composition such that the flow of the composition on the dipping pin was significantly reduced. However, it was discovered that the addition of these gelling agents reduced the capsule's ability to withstand temperatures sufficiently high enough for vaporization of various vaporizable materials.

Applicant also attempted to expedite or encourage more rapid curing or setting of the composition after deposition of the composition onto the dipping pin by thermal cooling and thermal heating methods.

Applicant's problem with the composition of HPMC-CNC was that it demonstrated Newtonian behavior, and that its viscosity remained linearly proportional to the strain rate or shear stress applied thereon. Applicant understood that the problem of the sloughing of the composition was a result of the low viscosity of the composition during the dipping process, which is required to permit dipping. A person of ordinary skill would understand that it is difficult to dip into a pool of a composition if the composition has high viscosity. That is, the viscosity of the composition required during the dipping process should sufficiently low enough to permit sloughing. However, in order to prevent sloughing of the material after the composition was deposited onto the dipping pin, a change or increase in viscosity to a viscosity level sufficient to prevent sloughing was required immediately after the dipping process. Applicant's earlier attempts to increase the viscosity of the composition immediately after the dipping process included introducing gelling agents, and use of various known thermal heating or cooling methods to increase the viscosity of the composition once the composition was deposited onto the dipping pin.

An alternate method for increasing a viscosity of the composition immediately after the dipping process was to take advantage of the behavior of non-Newtonian fluids. More specifically, Applicant desired to take advantage of the properties of a colloidal shear thinning fluid. Applicant notes that a shear thinning fluid (also known as a pseudoplastic fluid) is a non-Newtonian fluid where its viscosity increases when a shear force is not applied to it, and where its viscosity decreases when a shear force is applied thereon. Such a system would allow the pool of capsule forming composition to have a low viscosity, as a continual shearing force can be applied thereto by stirring or other means. Following the dipping or submerging process and when the dipping pin is removed from the pool, the capsule forming composition would no longer be subjected to any shearing force, which would result in an increase in viscosity sufficient to prevent sloughing of the composition during the curing process.

After many attempts, Applicant found that the addition of a shear thinning additive, such as an alcohol (for example isopropyl alcohol) changed the behavior of the composition to exhibit non-Newtonian fluid dynamics, and more specifically exhibit colloidal shear thinning fluid properties. Upon the addition of the shear thinning additive (for example isopropyl alcohol) the composition's viscosity would increase sufficiently when a shear force was removed and an amount of sloughing was significantly reduced during the curing process. Tests conducted demonstrated that the addition of any amount of the shear thinning additive started to change the behavior of the composition to exhibit non-Newtonian fluid dynamics. Applicant notes that HPMC is a non-ionic cellulose ether and is soluble in water. Accordingly, any solvent for the dissolution of the mixture of HPMC:CNC should contain sufficient amounts of water and the shear thinning additive. Thus, in embodiments, the solvent having at least a 1% vol. of the shear thinning additive would exhibit non-Newtonian fluid dynamic behavior. In a preferred embodiment, a 1:2 vol:vol ratio of isopropyl alcohol to water yielded good results. Additionally, Applicant notes that the addition of isopropyl alcohol to water also had additional advantages of having anti-microbial properties.

Initially, isopropyl alcohol was added in an attempt to influence the thermal setting properties of the composition, however such attempts were met with limited success. Upon examining the composition with the added isopropyl alcohol, Applicant discovered that the viscosity of the undisturbed liquid had increased substantially. However upon stirring, Applicant found that the viscosity was significantly reduced, only to increase again shortly after stirring was ceased.

Applicant discovered that for Applicant's purpose, a composition of 4:1 HPMC:CNC dissolved in a solvent solution of 1:2 (vol:vol) of isopropyl alcohol:water produced a capsule having optimal physical characteristics. In another embodiment, a ratio of HPMC:CNC of 7:3 also produced a capsule having effective physical characteristics.

As discussed above, different physical characteristics can be elucidated and be taken advantage of by differing the combination and/or ratio of the HPMC:CNC mixture used to make the capsule forming composition. For example, and as aforementioned, a HPMC:CNC ratio of 4:1 produces a capsule having an ability to withstand higher temperatures. A HPMC:CNC ratio of 7:3 produces a capsule having a little more structural integrity but with increased brittleness after being subjected to temperatures as high as a capsule with a 4:1 ratio of HPMC:CNC. Accordingly, in order to take advantage of both strengths, and in an embodiment, a laminate capsule can be manufactured by multiple dippings in two or more different dipping compositions which can optionally be repeated in varying dipping orders.

More specifically, and as an example, a first layer of the capsule can be formed by at least partially submerging the dipping pin in a pool of a composition having a 4:1 ratio of HPMC:CNC. Subsequent to drying the first layer, the dipping pin can be submerged in a second pool of composition, such as a pool of a composition having a 7:3 ratio of HMPC:CNC, and then allowed to dry. By this process of layering with different compositions, the laminate capsule can have a large variety of physical characteristics, which can be customized to suit the needs of an application of the final capsule formed.

As discussed above, traditional methods of dip molding require that after a capsule is formed by depositing the composition onto the dipping pin, and curing the composition for forming the capsule, the capsule must be removed from the dipping pin by sliding or otherwise removing the formed capsule off the dipping pin. In an embodiment, a formed capsule can be gripped and then lateral forces can be applied to simply remove the capsule from the dipping pin. In another embodiment, a push pin can be inserted into the dipping pin to encourage the removal of the formed capsule. However, this process can be difficult and can require complicated machinery and/or release agents to successfully remove the capsule without damaging the capsule. This process also generally requires the use of a draft angle on the dipping pin, and the absence of a draft angle on the dipping pin can make the removal of the formed capsule extremely difficult. This process can also lead to a high number of rejected capsules that do not meet strict production standards.

While engaged in the development of vaporizable capsules, it became apparent that there was a major challenge in removing formed capsules from the dipping pins without damaging the formed capsules, even using push pins. Initially stainless steel pins were used to form the capsules, but it was discovered to be difficult or impossible to remove the formed capsules from the dipping pins without causing damage to the capsules unless a draft angle of at least 3 degrees was utilized on the dipping pins.

Subsequently, coating the stainless steel pins with a release agent or lubricant prior to the deposition of the capsule-forming material onto the dipping pin was attempted. Initial attempts were made by coating a stainless steel dipping pin with a lubricant such as demolding oil or other generally known mold release agents. However, coating the stainless steel pins with lubricants was met with limited success. Further attempts were made by depositing low-friction materials such as Teflon^{®} and diamond like coatings (DLC) directly onto the surface of the pins. However, coating the stainless steel pins with lubricants and/or low-friction coatings were met with limited success. Applicant noted that while the lubricant and/or low-friction coatings reduced the friction between the capsule and the stainless steel dipping pin, the reduction of friction was insufficient to overcome the adherence forces acting between the capsule and the dipping pin.

Applicant continued to conduct experiments on methods of reducing the friction acting between the capsule and the dipping pin, such that a completed capsule can be relatively easily removed from the dipping pin without causing damage thereto.

Another method for reducing the friction acting between the capsule and the dipping pin was to introduce a draft angle on the dipping pin. That is, if the dipping pin was slightly tapered, there would only be one point of contact between the capsule and the dipping pin, such that the frictional forces that must be overcome to remove the capsule from the dipping pin would be greatly reduced. This, however, results in a capsule that is slightly tapered (having a draft angle corresponding to the draft angle of the dipping pin) and also having a single point of contact for sealing purposes. Desirably, Applicant wanted a capsule without any draft angles.

Adjustments to the surface finish of the dipping pin to make the surface smoother were also attempted to assist in the removal of the capsules from the dipping pin, but this did not work well.

In several attempts, dipping pins were made out of various materials having low surface adhesion (such as Teflon^{®}). But again, this did not produce a notable improvement in the removal of the formed capsules from the dipping pin.

After many experiments, Applicant manufactured a dipping pin using an elastomeric material and discovered that a dipping pin made of an elastomeric material can shrink radially (in diameter) when it is stretched in the axial or longitudinal direction. The radial shrinking of the elastomeric pin caused an exterior surface of the elastomeric pin to separate from the interior surface of the formed capsule, which simplified the removal of the capsule from the dipping pin. After a first trial run, experiments with a number of different elastomeric materials were conducted and it was discovered that elastomeric materials have the potential to work in assisting with the removal of the capsule from the capsule forming pins, and that different elastomers (or combinations thereof) can be selected based on desired principles such as chemical resistance and longevity.

The experiments conducted also experimented with different durometers of elastomeric materials and found that the durometer of the elastomeric material effects the removal of the formed capsule from the dipping pin. It was discovered that by making the dipping pin from an elastomeric material (such as silicone), it is possible to elongate the elastomeric dipping pin in an axial or longitudinal direction, thereby radially reducing a diameter of the elastomeric dipping pin, and causing separation of the capsule from the dipping pin.

The elastomeric dipping pin may be comprised of only elastomeric material (or a combination of different elastomeric materials) or may be comprised of a combination of elastomeric and non-elastomeric materials. The elastomeric dipping pin may also contain a hollow or annular cavity (which may be open at one or both ends) which further helps to reduce the diameter of the capsule-forming pin during elongation in the linear direction. Additionally, a non-elastomeric material, such as a push pin, may be inserted into the annular cavity in the elastomeric pin to linearly stretch the elastomeric pin to radially reduce the diameter of the elastomeric pin, thereby facilitating the easy removal of the formed capsule from the elastomeric pin. The elastomeric material may only comprise a portion of the exterior surface of the dipping pin, with other sections of the dipping pin being made from other materials such as stainless steel.

Additionally, the elastomeric dipping pin can permit manufacture of pins to form various capsule shapes or other shapes that would otherwise be impossible to form difficult or impossible to remove from a non-elastomeric pin. These capsules or other shapes could include a significant undercut on the elastomeric pin, permitting the forming of other capsule shapes or other shapes which wouldn't be able to be removed from a non-elastomer pin because the undercut would be smaller than the rest of the body of the pin.

Applicant came to understand that by employing an elastomeric dipping pin, it is possible to radially reduce the diameter of the elastomeric dipping pin which can allow novel capsule shapes or other shapes to be formed and successfully removed from the elastomeric pins. For example, a capsule that is shaped like 3/4 of a sphere can be formed. Normally such a shaped capsule would be very difficult or impossible to remove from a non-elastomeric pin because the opening in the top of the sphere would be smaller than the mid-section of the sphere and so the formed 3/4 sphere capsule couldn't be removed from the pin unless you have a way to radially reduce the diameter of the pin. Further, shapes such as squares, rectangles, triangles, and other shapes with sharp angles are generally very difficult to remove from traditional non-elastomer pins, but those shapes could easily be formed and removed using an elastomeric dipping pin system.

In embodiments, the elastomeric pin can be an elastomeric rod or tube which can be used to form hollow tube-like structures by dipping into or coating a section of the elastomeric rod or tubing with the capsule forming solution and subsequently curing the capsule forming solution. After curing, the formed hollow tube can be removed from the elastomeric rod or tube by axial stretching of the elastomeric rod or tube, thereby radially reducing the diameter of the elastomeric rod or tube, and permitting the formed hollow tube to be removed from the elastomeric rod or tube. The resulting formed hollow tube structure can be advantageously used as components in the construction of vaporizable capsules.

The capsule forming substances can also be applied to all of or a portion of the surface of the elastomeric dipping pins by means other than dipping, such as by spraying, electrostatic deposition, painting, transferring, or other means of applying capsule forming material onto the surface of the elastomeric pin.

In an embodiment, an elastomeric dipping pin incorporating an annular cavity could also be shrunk or reduced in size by applying negative air pressure on an interior of the pin for removing the formed capsule. In an alternate, but similar embodiment, the interior of the elastomeric pin can be inflated with positive pressure while dipping and after dipping is completed, the air can be released from the cavity, thereby collapsing the annular cavity and separating the dipping pin from the formed capsule so the dipping pin can be removed from the formed cavity. Alternatively, the capsule could have an internal structure that serves to reduce the outside diameter of the pin, such as a collapsible metal structure attached to the inside wall of the annular cavity of the elastomeric dipping pin.

Also, an elastomeric pin could be used by stretching/expanding a hollow inner cavity of the pin (either mechanically or by air pressure or the like) prior to dipping in the dipping pool and then relaxed/contracting the elastomeric pin after the dipping to form capsules with grooves and other structures that would otherwise be difficult or impossible to form because the dipping liquid would not be able to flow into the grooves or other structures in the unexpanded form of the elastomeric pin. It is known that non-elastomeric materials cannot be used to form such shaped capsules because the capsule-forming material doesn't want to flow into small grooves, as such a process typically traps air pockets in the grooves between the outer surface of the pin and the inner surface of the formed capsule.

Repeated use of an elastomeric dipping pin resulted in a gradual decrease in the structural integrity of the elastomeric material. The constant stretching in the axial direction resulted in an elastomeric pin that became deformed due to the loss of elasticity of the material, and the elastomeric pin did not return to its original shape and size. To correct for this gradual loss of elasticity, elastomeric materials having higher elasticity were used, as well as high grade memory form type materials, but the gradual loss of the elasticity of the elastomeric material was continually observed.

Accordingly, Applicant positioned a supporting pin within the elastomeric dipping pin to provide additional support to the elastomeric dipping pin. The supporting pin provides external structural rigidity to assist in preventing deformation of the elastomeric dipping pin during the dipping process and axial stretching, and also assists with the maintaining of the dipping pin's original shape and structure, even after repeated axial stretching. In an embodiment, the supporting pin can be positioned concentrically within an annular cavity of the dipping pin. In an embodiment, the support pin can also function as the push pin when removing the formed capsule from the dipping pin. In an embodiment, a portion of the supporting pin can be joined to a section of the annular cavity to prevent linear stretching in a desired section of the elastomeric pin.

In order to position a support pin within an annular cavity of an elastomeric dipping pin, Applicant found that the use of a lubricant, such as silicone, propylene glycol or lithium grease greatly reduces frictional contact therebetween. However, a clearance between the interior wall of the annular cavity of the elastomeric dipping pin and the exterior surface of the supporting pin is sufficiently low than any lubricant added therebetween can be forced out after only a few uses. Accordingly, grease or lubricant grooves, dimples, apertures, and the like can be disposed on either of the interior surface of the annular cavity or the exterior surface of the supporting pin for storing an amount of the lubricant. The grooves, dimples, apertures and the like maintain high surface area contact and still permits space for the lubricant such that the lubricant is not forced out after a single use. In another embodiment, the supporting pin can be hollow (ie. annulus) with holes or channels designed to allow the lubricant to flow in and out, or otherwise communicate from the annulus into the annular space between the annular cavity and the exterior surface of the supporting pin.

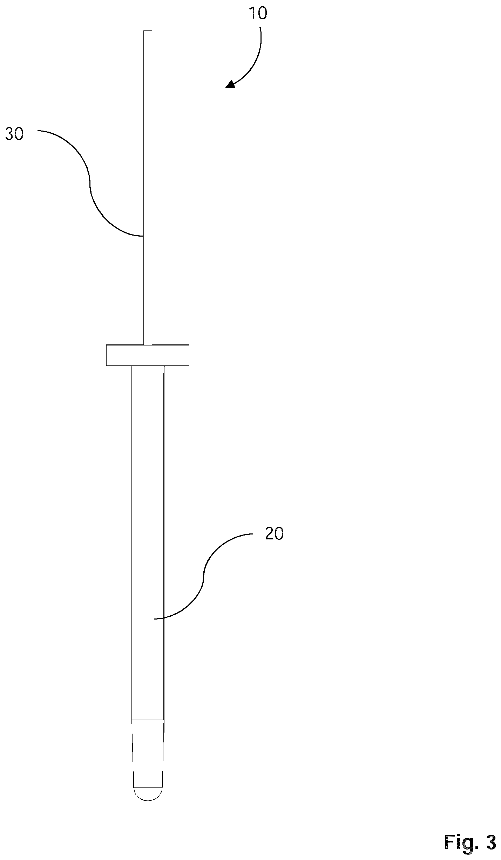

With reference to Fig. 3, a molding pin 10 comprises an elastomeric dipping pin 20 having a supporting pin 30 positioned therein. As discussed above, in an embodiment, the supporting pin 30 is positioned within the elastomeric dipping pin 20, and the pins 20,30 can be submerged or dipped into a pool of capsule-forming composition for depositing a layer of the composition onto an exterior surface of the dipping pin 20. The pins 20,30 are then retracted or pulled out of the pool of the composition and the composition is permitted to dry and cure. Once the composition has fully cured, the formed capsule can be removed from the dipping pin 20 and then stored for future use. A person skilled in the art would understand that in order for the supporting pin 30 to be positioned within the dipping pin 20, the dipping pin 20 should have an annular cavity 40 for accepting the supporting pin 30 therein.

As discussed above, in an embodiment, lubricant grooves can be disposed on either or both of an exterior surface of the supporting pin 30 or on an interior surface or wall of an annular cavity 40 formed in the elastomeric dipping pin 20.

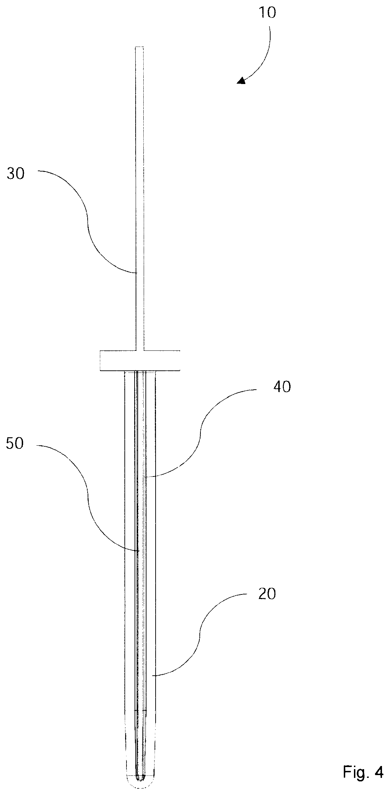

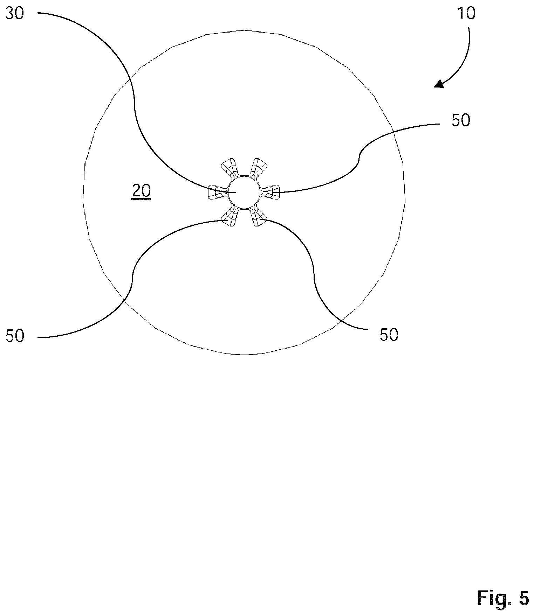

With specific reference to Figs. 4 and 5, the elastomeric dipping pin 20 can further comprises the annular cavity 40 having an interior surface, which can further comprise at least one lubricant groove or structure 50.

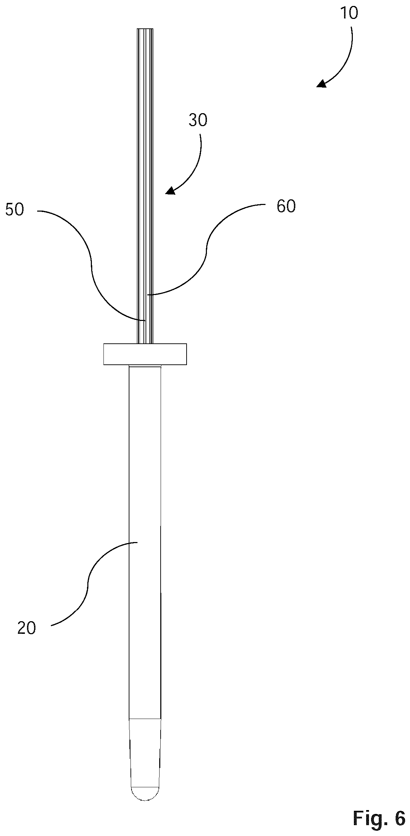

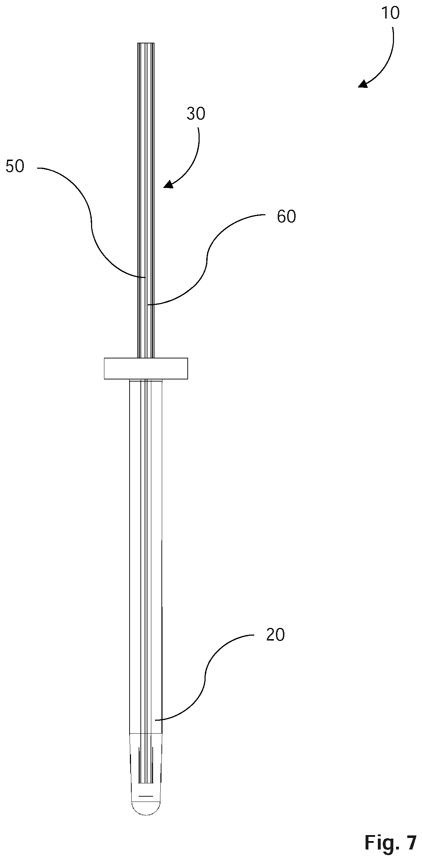

Alternatively, in another embodiment, and as discussed above, the lubricant grooves or structures can be disposed on an exterior surface of the supporting pin 30. With reference to Figs. 6 and 7, the supporting pin 30 can further comprise the at least one lubricant groove or structure 50 on an exterior surface thereof.

Although not shown, in embodiments, in order to ensure consistency in the depth of the supporting pin 30 being positioned within the annular cavity 40 of the dipping pin 20, a stop can be placed at a bottom of the annular cavity 40. The stop can be any solid piece of material, even the same material as the supporting pin. In an alternate embodiment, a dab of adhesive can be placed at the bottom of the annular cavity and the supporting pin, having a threaded top portion can be positioned therein. After the adhesive cures, the threaded supporting pin can be unthreaded and removed to leave a corresponding threaded stop. Lubricant or grease can be applied to the supporting pin in the usual manner and then the lubricated supporting pin can be placed with the annular cavity.

In another embodiment, the supporting pin can be a tube having its own annular cavity for accepting lubricant therein. In this embodiment, the supporting pin can further comprise apertures for allowing lubricant within the supporting pin's annular cavity to flow therethrough and in between the supporting pin and the elastomeric dipping pin. In such an embodiment, the lubricant grooves are obviated.

## Claims

1. A capsule forming composition, for manufacturing a capsule for use in a vaporizer, the capsule forming composition comprising a mixture of:
hydroxypropyl-methyl cellulose (HPMC); and
cellulose nanocrystals (CNC), a ratio of HPMC:CNC being between 4:1 to 7:3, the mixture dissolved in a solvent solution having a 1:2 ratio of isopropyl alcohol to water.

2. A method of manufacturing a capsule, the method comprising:
providing an elastomeric dipping pin having an annular cavity;
providing a supporting pin;
positioning the supporting pin within the annular cavity of the dipping pin;
depositing at least one layer of a capsule forming composition onto an exterior surface of the dipping pin, the capsule forming composition including a mixture of hydroxypropyl-methyl cellulose (HPMC) and cellulose nanocrystals (CNC), a ratio of HPMC:CNC being between 4:1 to 7:3, the mixture dissolved in a solvent solution having a 1:2 ratio of isopropyl alcohol to water;
curing the at least one layer of capsule forming composition for forming a capsule; and
removing the formed capsule from the dipping pin.

3. The method of claim 2, further comprising lubricating between the dipping pin and the supporting pin.

4. The method of claim 2 or 3, wherein depositing at least one layer of a capsule forming composition onto the exterior surface of the dipping pin further comprises at least partially submerging the dipping pin having the supporting pin positioned therein into a pool of the capsule forming composition.

5. The method of claim 2, 3, or 4, wherein curing the at least one layer of capsule forming composition further comprises removing the dipping pin coated with the capsule forming composition from a pool of capsule forming composition and allowing it to dry.

6. The method of any one of claims 2 to 5, wherein removing the formed capsule from the dipping pin further comprises pushing the supporting pin further into the annular cavity for axially extending the dipping pin, thereby reducing a diameter of the dipping pin to cause separation between the formed capsule and the exterior surface of the dipping pin.

7. The method of any one of claims 2 to 6, further comprising repeating the steps of depositing at least one layer of capsule forming composition and curing the at least one layer of capsule forming composition to create a capsule having multiple layers of the capsule forming composition.

8. A molding pin for manufacturing a capsule from a capsule forming composition, the molding pin comprising a dipping pin having an elastomeric portion on which the capsule forming composition is deposited thereon, the capsule forming composition including a mixture of hydroxypropyl-methyl cellulose (HPMC) and cellulose nanocrystals (CNC), a ratio of HPMC:CNC being between 4:1 to 7:3, the mixture dissolved in a solvent solution having a 1:2 ratio of isopropyl alcohol to water.

9. The molding pin of claim 8, further comprising a supporting pin.

10. The molding pin of claim 9, wherein the dipping pin further comprises an annular cavity for accepting the supporting pin therein.

11. The molding pin of claim 10, wherein the annular cavity further comprises one or more lubricant grooves for storing a lubricant.

12. The molding pin of claim 10, wherein the supporting pin further comprises one or more lubricant grooves disposed on an exterior surface thereof for storing a lubricant.

13. The molding pin of claim 10, wherein the annular cavity further comprises one or more lubricant grooves for storing a lubricant and the supporting pin further comprises one or more lubricant grooves disposed on an exterior surface thereof for storing the lubricant.

14. The molding pin of any one of claims 9 to 13, wherein the supporting pin further comprises an annulus and one or more lubricant passageways for communicating a lubricant from the annulus into an annular space between the dipping pin and an exterior surface of the supporting pin.

## Patentansprüche

1. Kapselbildende Zusammensetzung zum Herstellen einer Kapsel zur Verwendung in einem Vaporizer, wobei die kapselbildende Zusammensetzung eine Mischung umfasst von:
Hydroxypropylmethylcellulose (HPMC); und
Cellulose-Nanokristallen (CNC), wobei ein Verhältnis von HPMC:CNC zwischen 4:1 und 7:3 liegt, wobei die Mischung, die in einer Lösungsmittellösung gelöst ist, ein Verhältnis von Isopropylalkohol zu Wasser von 1:2 aufweist.

2. Verfahren zum Herstellen einer Kapsel, wobei das Verfahren Folgendes umfasst:
Bereitstellen eines elastomeren Tauchstifts, der einen ringförmigen Hohlraum aufweist;
Bereitstellen eines Stützstifts;
Positionieren des Stützstifts innerhalb des ringförmigen Hohlraums des Tauchstifts;
Absetzen mindestens einer Schicht einer kapselbildenden Zusammensetzung auf eine Außenfläche des Tauchstifts, wobei die kapselbildende Zusammensetzung eine Mischung von Hydroxypropylmethylcellulose (HPMC) und Cellulose-Nanokristallen (CNC) einschließt, wobei ein Verhältnis von HPMC:CNC zwischen 4:1 und 7:3 liegt, wobei die Mischung, die in einer Lösungsmittellösung gelöst ist, ein Verhältnis von Isopropylalkohol zu Wasser von 1:2 aufweist;
Aushärten der mindestens einen Schicht kapselbildender Zusammensetzung zum Bilden einer Kapsel; und
Entfernen der gebildeten Kapsel von dem Tauchstift.

3. Verfahren nach Anspruch 2, ferner das Gleitfähigmachen zwischen dem Tauchstift und dem Stützstift umfassend.

4. Verfahren nach Anspruch 2 oder 3, wobei das Absetzen mindestens einer Schicht einer kapselbildenden Zusammensetzung auf der Außenfläche des Tauchstifts ferner das mindestens teilweise Untertauchen des Tauchstifts, in dem der Stützstift positioniert ist, in ein Reservoir der kapselbildenden Zusammensetzung umfasst.

5. Verfahren nach Anspruch 2, 3 oder 4, wobei das Aushärten der mindestens einen Schicht kapselbildender Zusammensetzung ferner das Entfernen des Tauchstifts, der mit der kapselbildenden Zusammensetzung beschichtet ist, aus einem Reservoir kapselbildender Zusammensetzung und das Gestatten derselben, zu trocknen, umfasst.

6. Verfahren nach einem der Ansprüche 2 bis 5, wobei das Entfernen der gebildeten Kapsel von dem Tauchstift ferner das Schieben des Stützstifts weiter in den ringförmigen Hohlraum zum axialen Verlängern des Tauchstifts umfasst, wodurch ein Durchmesser des Tauchstifts reduziert wird, um eine Trennung zwischen der gebildeten Kapsel und der Außenfläche des Tauchstifts zu verursachen.

7. Verfahren nach einem der Ansprüche 2 bis 6, ferner das Wiederholen der Schritte des Absetzens mindestens einer Schicht kapselbildender Zusammensetzung und des Aushärtens der mindestens einen Schicht kapselbildender Zusammensetzung umfassend, um eine Kapsel zu erzeugen, die mehrere Schichten der kapselbildenden Zusammensetzung aufweist.

8. Formstift zum Herstellen einer Kapsel aus einer kapselbildenden Zusammensetzung, wobei der Formstift einen Tauchstift umfasst, der einen elastomeren Abschnitt aufweist, auf den die kapselbildende Zusammensetzung abgesetzt wird, wobei die kapselbildende Zusammensetzung eine Mischung von Hydroxypropylmethylcellulose (HPMC) und Cellulose-Nanokristallen (CNC) einschließt, wobei ein Verhältnis von HPMC:CNC zwischen 4:1 und 7:3 liegt, wobei die Mischung, die in einer Lösungsmittellösung gelöst ist, ein Verhältnis von Isopropylalkohol zu Wasser von 1:2 aufweist.

9. Formstift nach Anspruch 8, der ferner einen Stützstift umfasst.

10. Formstift nach Anspruch 9, wobei der Tauchstift ferner einen ringförmigen Hohlraum zum Aufnehmen des Stützstifts darin umfasst.

11. Formstift nach Anspruch 10, wobei der ringförmige Hohlraum ferner eine oder mehrere Gleitmittelkerben zum Speichern eines Gleitmittels umfasst.

12. Formstift nach Anspruch 10, wobei der Stützstift ferner eine oder mehrere Gleitmittelkerben umfasst, die auf einer Außenfläche davon zum Speichern eines Gleitmittels angeordnet sind.

13. Formstift nach Anspruch 10, wobei der ringförmige Hohlraum ferner eine oder mehrere Gleitmittelkerben zum Speichern eines Gleitmittels umfasst und der Stützstift ferner eine oder mehrere Gleitmittelkerben umfasst, die auf einer Außenfläche davon zum Speichern des Gleitmittels angeordnet sind.

14. Formstift nach einem der Ansprüche 9 bis 13, wobei der Stützstift ferner einen Ring und einen oder mehrere Gleitmitteldurchgänge zum Übertragen eines Gleitmittels von dem Ring in einen ringförmigen Raum zwischen dem Tauchstift und einer Außenfläche des Stützrings umfasst.

## Revendications

1. Composition de formation de capsule, pour fabriquer une capsule pour l'utilisation dans un vaporisateur, la composition de formation de capsule comprenant un mélange :
d'hydroxypropylméthyl cellulose (HPMC) ; et
de nanocristaux de cellulose (CNC), un ratio de la HPMC :CNC se situant entre 4 :1 à 7 :3, le mélange dissous dans une solution de solvant ayant un ratio de 1 :2 de l'alcool d'isopropyle à l'eau.

2. Procédé de fabrication d'une capsule, le procédé comprenant les étapes consistant à :
fournir une broche d'immersion élastomère ayant une cavité annulaire ;
fournir une broche de support ;
positionner la broche de support à l'intérieur de la cavité annulaire de la broche d'immersion ;
déposer au moins une couche d'une composition de formation de capsule sur une surface extérieure de la broche d'immersion, la composition de formation de capsule incluant un mélange d'hydroxypropylméthyl cellulose (HPMC) et de nanocristaux de cellulose (CNC), un ratio de la HPMC :CNC étant situé entre 4 :1 à 7 :3, le mélange dissous dans une solution de solvant ayant un ratio de 1 :2 de l'alcool d'isopropyle à l'eau ;
durcir l'au moins une couche de la composition de formation de capsule pour former une capsule ; et
retirer la capsule formée de la broche d'immersion.

3. Procédé selon la revendication 2, comprenant en outre la lubrification entre la broche d'immersion et la broche de support.

4. Procédé selon la revendication 2 ou 3, dans lequel le dépôt d'au moins une couche d'une composition de formation de capsule sur la surface extérieure de la broche d'immersion comprend en outre le fait de plonger au moins partiellement la broche d'immersion ayant la broche de support positionnée en son sein dans un bassin de la composition de formation de capsule.

5. Procédé selon la revendication 2, 3, ou 4, dans lequel le durcissement de l'au moins une couche de composition de formation de capsule comprend en outre le retrait de la broche d'immersion revêtue de la composition de formation de capsule d'un bassin de composition de formation de capsule et le fait de la laisser sécher.

6. Procédé selon l'une quelconque des revendications 2 à 5, dans lequel le retrait de la capsule formée de la broche d'immersion comprend en outre le fait de pousser la broche de support plus loin dans la cavité annulaire pour l'extension axialement de la broche d'immersion, réduisant ainsi un diamètre de la broche d'immersion pour entraîner une séparation entre la capsule formée et la surface extérieure de la broche d'immersion.

7. Procédé selon l'une quelconque des revendications 2 à 6, comprenant en outre la répétition des étapes de dépôt d'au moins une couche de composition de formation de capsule et de durcissement de l'au moins une couche de composition de formation de capsule pour créer une capsule ayant plusieurs couches de la composition de formation de capsule.

8. Broche de moulage pour fabriquer une capsule à partir d'une composition de formation de capsule, la broche de moulage comprenant une broche d'immersion ayant une portion élastomère sur laquelle la composition de formation de capsule est déposée, la composition de formation de capsule incluant un mélange d'hydroxypropylméthyl cellulose (HPMC) et de nanocristaux de cellulose (CNC), un ratio de la HPMC :CNC étant situé entre 4 :1 à 7 :3, le mélange dissous dans une solution de solvant ayant un ratio de 1 :2 de l'alcool d'isopropyle à l'eau.

9. Broche de moulage selon la revendication 8, comprenant en outre une broche de support.

10. Broche de moulage selon la revendication 9, dans laquelle la broche d'immersion comprend en outre une cavité annulaire pour accepter en son sein la broche de support.

11. Broche de moulage selon la revendication 10, dans laquelle la cavité annulaire comprend en outre une ou plusieurs rainures de lubrifiant pour stocker un lubrifiant.

12. Broche de moulage selon la revendication 10, dans laquelle la broche de support comprend en outre une ou plusieurs rainures de lubrifiant disposées sur une surface extérieure de celle-ci pour stocker un lubrifiant.

13. Broche de moulage selon la revendication 10, dans laquelle la cavité annulaire comprend en outre une ou plusieurs rainures de lubrifiant pour stocker un lubrifiant et la broche de support comprend en outre une ou plusieurs rainures de lubrifiant disposées sur une surface extérieure de celle-ci pour stocker le lubrifiant.

14. Broche de moulage selon l'une quelconque des revendications 9 à 13, dans laquelle la broche de support comprend en outre un anneau et une ou plusieurs voies de passage de lubrifiant pour faire communiquer un lubrifiant depuis l'anneau dans un espace annulaire entre la broche d'immersion et une surface extérieure de la broche de support.
